(19) Europäisches Patentamt
European Patent Office
Office européen des brevets

(11) **EP 1 994 284 B1**

(12) **EUROPÄISCHE PATENTSCHRIFT**

(45) Veröffentlichungstag und Bekanntmachung des Hinweises auf die Patenterteilung:
**04.05.2011 Patentblatt 2011/18**

(21) Anmeldenummer: **07711730.7**

(22) Anmeldetag: **01.03.2007**

(51) Int Cl.:
**F04B 43/12** *(2006.01)* **F04B 43/08** *(2006.01)*
**F04B 49/10** *(2006.01)*

(86) Internationale Anmeldenummer:
**PCT/EP2007/001754**

(87) Internationale Veröffentlichungsnummer:
**WO 2007/104435 (20.09.2007 Gazette 2007/38)**

(54) **VERFAHREN UND VORRICHTUNG ZUM BETREIBEN EINER ELEKTRISCHEN PERISTALTISCHEN SCHLAUCHPUMPE**

METHOD AND DEVICE FOR OPERATING AN ELECTRIC PERISTALTIC HOSE PUMP

PROCÉDÉ ET DISPOSITIF POUR FAIRE FONCTIONNER UNE POMPE PERISTALTIQUE ÉLECTRIQUE

(84) Benannte Vertragsstaaten:
**AT BE BG CH CY CZ DE DK EE ES FI FR GB GR HU IE IS IT LI LT LU LV MC MT NL PL PT RO SE SI SK TR**

(30) Priorität: **11.03.2006 DE 102006011346**

(43) Veröffentlichungstag der Anmeldung:
**26.11.2008 Patentblatt 2008/48**

(73) Patentinhaber: **Fresenius Medical Care Deutschland GmbH**
**61352 Bad Homburg v.d.H. (DE)**

(72) Erfinder: **KOPPERSCHMIDT, Pascal**
**97456 Dittelbrunn (DE)**

(74) Vertreter: **Luderschmidt, Schüler & Partner Patentanwälte**
**John-F.-Kennedy-Strasse 4**
**65189 Wiesbaden (DE)**

(56) Entgegenhaltungen:
**WO-A-93/12827**  **WO-A-94/20157**
**WO-A-03/072942**  **US-A- 5 629 871**

**Beschreibung**

Die Erfindung bezieht sich auf ein Verfahren und eine Vorrichtung zum Betreiben einer elektrischen peristaltischen Schlauchpumpe zur Förderung einer Flüssigkeit in einer Schlauchleitung. Darüber hinaus betrifft die Erfindung ein Verfahren zum Betreiben einer elektrischen peristaltischen Schlauchpumpe einer extrakorporalen Blutbehandlungsvorrichtung sowie eine extrakorporale Blutbehandlungsvorrichtung mit einer Vorrichtung zum Betreiben einer peristaltischen Schlauchpumpe.

Bei einer extrakorporalen Blutbehandlung, beispielsweise einer Hämodialyse, durchströmt das zu behandelnde Blut in einem extrakorporalen Blutkreislauf die Blutkammer eines durch eine semipermeable Membran in die Blutkammer und eine Dialysierflüssigkeitskammer unterteilten Dialysators, während in einem Dialysierflüssigkeitssystem Dialysierflüssigkeit die Dialysierflüssigkeitskammer durchströmt. Der extrakorporale Blutkreislauf weist eine arterielle Schlauchleitung auf, die zu der Blutkammer führt, und eine venöse Schlauchleitung auf, die von der Blutkammer abgeht. Die bekannten Hämodialysevorrichtungen verfügen über eine Blutpumpe, die im Allgemeinen stromauf der Blutkammer des Dialysators angeordnet ist, um einen ausreichenden Blutfluss im extrakorporalen Blutkreislauf sicherzustellen.

An die Blutpumpe werden hohe technische Anforderungen gestellt. Daher kommen nur bestimmte Pumpentypen in Betracht. In der Praxis haben sich Schlauchpumpen bewährt, die das Blut des Patienten durch die arterielle und venöse Schlauchleitung fördern.

Die Schlauchpumpen werden nach ihrer Arbeitsweise auch als peristaltische Pumpen bezeichnet. Ihre Pumpwirkung basiert darauf, dass sich mindestens eine Verschlussstelle (Okklusion) längs der als Pumpenraum dienenden elastischen Schlauchleitung bewegt und dadurch die eingeschlossene Flüssigkeit in Förderrichtung verschiebt.

Bei der gebräuchlichsten Bauart der Schlauchpumpen erfolgt die Einstellung so, dass der elastische Schlauch an den bewegten Engstellen vollständig verschlossen wird. Daher werden diese Pumpen auch als okklusive Schlauchpumpen bezeichnet. Die beweglichen Eng- bzw. Verschlussstellen, die das Blut im Pumpenschlauch transportieren, können technisch auf verschiedene Weise erzeugt werden.

Es sind Rollenpumpen bekannt, bei denen der Schlauch zwischen einem Stator, der eine gebogene Rollenbahn als Widerlager bildet, und einem drehbar darin gelagerten, mit Rollen besetzten Rotor, eingefügt wird, so dass sich die Rollen in Förderrichtung auf dem Schlauch abwälzen. Vorzugsweise sind die Rollen federnd an dem Rotor gelagert, so dass sie auf den Schlauch eine Anpresskraft ausüben. Daneben sind auch Fingerpumpen bekannt, bei denen die Eng- bzw. Verschlussstellen durch eine längs des Schlauches angeordnete Reihe von beweglichen Stempeln (Fingern) erzeugt werden.

Einen Überblick über die unterschiedlichen Bauarten der Rollen- und Fingerpumpen wird in Dialysetechnik, 4. Auflage, Gesellschaft für angewandte Medizintechnik m.b.H. & Co. KG, Friedrichsdorf, 1988 gegeben.

Elektrische peristaltische Schlauchpumpen werden in den bekannten Blutbehandlungsvorrichtungen nicht nur zur Förderung des Blutes, sondern auch zur Förderung anderer Flüssigkeiten eingesetzt. An den ordnungsgemäßen Betrieb derartiger Schlauchpumpen bei der Verwendung in medizin-technischen Einrichtungen, insbesondere bei Blutbehandlungsvorrichtungen, werden hohe

Anforderungen gestellt.

Während des Betriebs der Rollenpumpen stellt sich das Problem, dass bei einer Erhöhung des Strömungswiderstandes eine vollständige Okklusion der Schlauchleitung nicht mehr gegeben ist. Vielmehr beginnen sich die Rollen von der Schlauchleitung abzuheben. Für diesen Fall ist ein ordnungsgemäßer Betrieb der Rollenpumpe nicht mehr sichergestellt.

Die US 5,629,871, beschreibt ein Verfahren und eine Vorrichtung zur Überwachung der Funktionsfähigkeit einzelner Baugruppen einer Hämodialysevorrichtung. Zu diesen zählen auch die Schlauchpumpen, wobei der Pumpenstrom oder die Spannung überwacht wird, um auf die Funktionsfähigkeit der Pumpe zu schließen. Aus der US 4,781,525 ist bekannt, den Pumpenstrom zur Bestimmung der Förderrate heranzuziehen.

Die WO 97/45150 beschreibt ein Verfahren zur Bestimmung des Förderdrucks einer Pumpe, bei dem der Pumpenstrom bestimmt wird. Da es unter bestimmen Umständen zu Abweichungen von einem linearen Zusammenhang zwischen Förderdruck und Pumpenstrom kommen kann, wird die Verwendung einer Kalibrierungskurve vorgeschlagen.

Der Erfindung liegt die Aufgabe zu Grunde, ein Verfahren und eine Vorrichtung zum Betreiben einer elektrischen peristaltischen Schlauchpumpe, insbesondere der Schlauchpumpe einer extrakorporalen Blutbehandlungsvorrichtung anzugeben, dass bzw. die eine sichere Überwachung des ordnungsgemäßen Betriebs der Schlauchpumpe erlaubt.

Diese Aufgabe wird mit den Merkmalen der Patentansprüche 1 und 9 gelöst. Vorteilhafte Ausführungsformen der Erfindung sind Gegenstände der Unteransprüche.

Der Erfindung liegt die Erkenntnis zu Grunde, dass bei vollständiger oder teilweiser Okklusion der Schlauchleitung durch die Verdrängerkörper einer elektrischen peristaltischen Pumpe, beispielsweise die Rollen einer Rollenpumpe, sich die Leistungsaufnahme der elektrischen Pumpe ändert. Die Erfindung beruht darauf, die von der Pumpe aufgenommene Leistung oder eine mit der Leistung korrelierende physikalische Größe, beispielsweise den von der Pumpe aufgenommen Strom zu bestimmen und aus der gemessenen physikalischen Größe einen Gleichanteil, der sich nicht periodisch ändert, und einen den Gleichanteil überlagernden Wechselanteil zu bestimmen, der sich periodisch ändert. Zur Überwachung des ordnungsgemäßen Betriebes der Schlauchpumpe wird während der Blutbehandlung überwacht, wie der Wechselanteil der Leistungsaufnahme im Verhältnis zum Gleichanteil der Leistungsaufnahme ansteigt bzw. abfällt. Auf einen nicht ordnungsgemäßen Betrieb der Schlauchpumpe wird dann auf der Grundlage des Gleichanteils und Wechselanteils, insbesondere bei einer charakteristischen zeitlichen Änderung des Gleichanteils und zeitlichen Änderung des Wechselanteils geschlossen.

In einer bevorzugten Ausführungsform wird die Leistungsaufnahme dadurch ausgewertet, dass die zeitliche Änderung des Gleichanteils und die zeitliche Änderung des Wechselanteils in vorgegebenen Zeitintervallen zueinander in ein Verhältnis gesetzt werden, wobei auf der Grundlage der zeitlichen Änderung des ermittelten Verhältnisses zwischen der zeitlichen Änderung des Gleichanteils und Wechselanteils in vorgegebenen Zeitintervallen während des Betriebs der Schlauchpumpe auf einen nicht ordnungsgemäßen Betrieb der Schlauchpumpe geschlossen wird.

Die Erfindung beruht insbesondere auf der Erkenntnis, dass der Quotient aus der zeitlichen Änderung des Wechselanteils und des Gleichanteils eine charakteristische Größe dafür ist, ob die Verdrängerkörper der peristaltischen Pumpe die Schlauchleitung vollständig oder nur teilweise okkludieren. Vorzugsweise wird der Quotient während der Blutbehandlung überwacht, um für den Fall, dass der Quotient einen bestimmten Grenzwert unterschreitet, darauf zu schließen, dass eine vollständige Okklusion nicht mehr gegeben ist. Je nach der Höhe des Grenzwertes kann das Maß der Okklusion festgelegt werden, bei dessen Unterschreiten der nicht ordnungsgemäße Betrieb der Pumpe angenommen wird. Vorteilhafterweise können auch mehrere Grenzwerte definiert werden, die schon den Beginn einer verschwindenden Okklusion erkennen lassen oder auf einen Störfall erst dann schließen lassen, wenn die Okklusion bereits zumindest teilweise aufgehoben ist.

Bei einer alternativen bevorzugten Ausführungsform wird nicht die zeitliche Änderung des Wechsel- und Gleichanteils in vorgegebenen Zeitintervallen, d.h. der Ableitung der Funktion oder der Steigung der Kurve, bestimmt, sondern der Quotient ($A_{AC}/I_{DC}$) des Wechselanteils (d/dt $A_{AC}$) und des Gleichanteils ($I_{DC}$) wird in vorgegebenen Zeitintervallen während des Betriebs der Schlauchpumpe berechnet und mit einem vorgegebenen Grenzwert verglichen, wobei auf eine nicht ausreichende Okklusion der Schlauchpumpe geschlossen wird, wenn der Quotient den vorgegebenen Grenzwert unterschreitet. Es kann aber auch der Quotient aus Gleich- und Wechselanteil bestimmt werden, wobei auf eine nicht ausreichende Okklusion der Schlauchpumpe geschlossen wird, wenn der Quotient den vorgegebenen Grenzwert überschreitet. Es können aber auch noch andere Terme für die Auswertung herangezogen werden, solange sie nur bei Beginn der Okklusion zu einer charakteristischen Änderung der Rechengröße führen.

Der Wechselanteil der gemessenen physikalischen Größe, die mit der Leistungsaufnahme der Pumpe korreliert, ist abhängig von der Bauart der Pumpe. Die Frequenz des Wechselanteils ist insbesondere abhängig von der Anzahl der Rollen der Rollenpumpe. Da die Frequenz des Rotors bekannt ist, kann der Wechselanteil leicht bestimmt werden. Hierzu bieten sich die bekannten mathematischen Verfahren an, zu denen beispielsweise die Fourieranalyse zählen.

Die erfindungsgemäße Vorrichtung verfügt über Mittel zum Bestimmen des Gleichanteils und Wechselanteils der Leistungsaufnahme bzw. der gemessenen physikalischen Größe, die mit der Leistungsaufnahme korreliert, und Mittel zur Feststellung eines nicht ordnungsgemäßen Betriebs der Schlauchpumpe. Die erfindungsgemäße Vorrichtung kann eine separate Baugruppe bilden oder Bestandteil der bekannten Blutbehandlungsvorrichtungen sein.

Als besonders vorteilhaft hat sich die Verwendung des erfindungsgemäßen Verfahrens und der Einsatz der erfindungsgemäßen Vorrichtung in extrakorporalen Blutbehandlungsvorrichtungen, beispielsweise Hämodialysevorrichtungen zur Überwachung des ordnungsgemäßen Betriebs der im extrakorporalen Blutkreislauf angeordneten Blutpumpe erwiesen. Mit dem erfindungsgemäßen Verfahren oder der erfindungsgemäßen Vorrichtung kann aber auch der Betrieb aller anderen Schlauchpumpen in medizin-technischen Einrichtungen oder im allgemeinen Maschinenbau überwacht werden.

Im Folgenden wird ein Ausführungsbeispiel der Erfindung unter Bezugnahme auf die Zeichnungen näher erläutert.

Es zeigen:

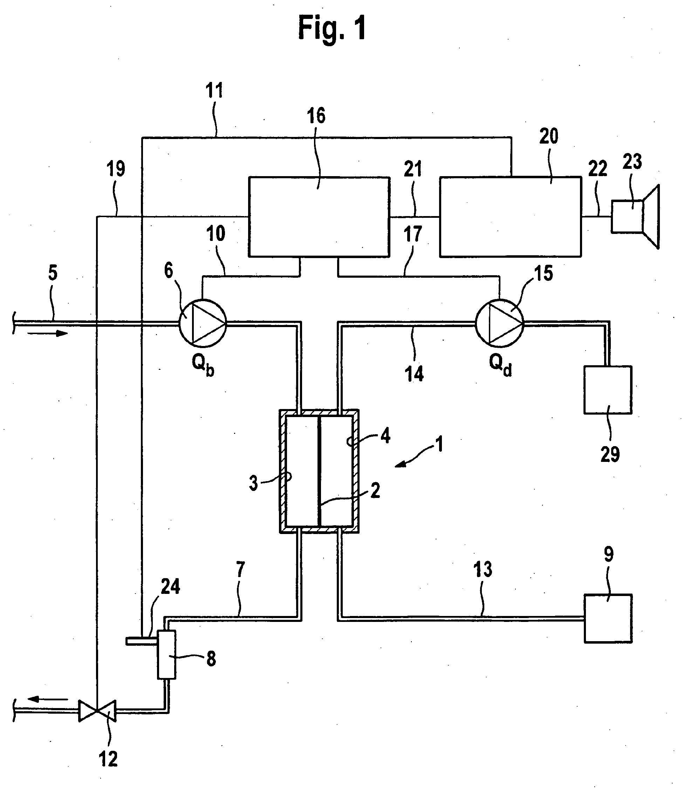

Figur 1        eine erfindungsgemäße extrakorporale Blutbehandlungsvorrichtung, die über eine erfindungsgemäße Vorrichtung zum Betreiben einer Schlauchpumpe der Blutbehandlungsvorrichtung verfügt,

Figur 2        der Gleich- und Wechselanteil der Leistungsaufnahme der Blutpumpe während einer Dialysebehandlung als Funktion der Behandlungsdauer, und

Figuren 3a bis 3c   die dynamischen Druckverhältnisse während des Betriebs einer Rollenpumpe bei einer vollständigen Okklusion, zu Beginn einer verschwindenden Okklusion und bei einer nicht vollständigen Okklusion der Schlauchleitung.

Die extrakorporale Blutbehandlungsvorrichtung, insbesondere Hämodialysevorrichtung, verfügt über einen Dialysator 1, der durch eine semipermeable Membran 2 in eine Blutkammer 3 und eine Dialysierflüssigkeitskammer 4 unterteilt ist. Von einem Patienten führt eine arterielle Blutleitung 5, in die eine Blutpumpe 6 geschaltet ist, zu einem Einlass der Blutkammer 3, während von einem Auslass der Blutkammer eine venöse Blutleitung 7 über eine Tropfkammer 8 zu dem Patienten führt. In der venösen Schlauchleitung 7 ist stromab der Tropfkammer 8 ein elektromagnetisch betätigbares Absperr-Ventil 12 angeordnet.

Die frische Dialysierflüssigkeit wird in einer Dialysierflüssigkeitsquelle 12 bereitgestellt. Von der Dialysierflüssigkeitsquelle 12 führt eine Dialysierflüssigkeitszuführleitung 13 zu einem Einlass der Dialysierflüssigkeitskammer 4 des Dialysators 1, während eine Dialysierflüssigkeitsabführleitung 14 von einem Auslass der Dialysierflüssigkeitskammer zu einem Abfluss 9 führt. In die Dialysierflüssigkeitsabführleitung 14 ist eine Dialysierflüssigkeitspumpe 15 geschaltet.

Bei der Blutpumpe 6 handelt es sich um elektrisch betriebene peristaltische Schlauchpumpe, insbesondere eine Rollenpumpe, wobei die arterielle und venöse Blutleitung 5, 7 flexible Schlauchleitungen sind, die in die Rollenpumpe 6 eingelegt werden. Bei dem venösen Absperr-Ventil 12 handelt es sich um elektromagnetisch betätigbare Schlauchklemme.

Die Dialysevorrichtung verfügt über eine Steuereinheit 16, die mit der Blutpumpe 6 und der Dialysierflüssigkeitspumpe 15 über Steuerleitungen 16, 17 verbunden ist. Die Steuereinheit 16 stellt eine bestimmte Spannung bzw. einen bestimmten Strom zum Betreiben der Blutpumpe 6 und der Dialysierflüssigkeitspumpe 12 zur Verfügung, so dass Blut in den Blutleitungen 5, 7 mit einer vorgegebenen Blutflussrate $Q_b$ und Dialysierflüssigkeit in den Dialysierflüssigkeitsleitungen 13, 14 mit einer vorgegebenen Dialysierflüssigkeitsrate $Q_d$ strömt. Darüber hinaus betätigt die Steuereinheit 16 über eine Steuerleitung 19 das venöse Absperrventil 12.

Neben der Steuereinheit 16 verfügt die Blutbehandlungsvorrichtung über eine Rechen- und Auswerteinheit 20, die über eine Datenleitung 21 mit der Steuereinheit 16 kommuniziert. Die Rechen- und Auswerteinheit 20 ist über eine weitere Datenleitung 22 mit einer Alarmeinheit 23 verbunden, die einen optischen und/oder akustischen Alarm gibt.

Zum Messen des Drucks in der venösen Blutleitung 7 ist ein venöser Drucksensor 24 an der Tropfkammer 8 angeordnet, der über eine Datenleitung 25 mit der Rechen- und Auswerteinheit 20 verbunden ist.

Die Dialysevorrichtung kann noch über weitere Komponenten, beispielsweise eine Bilanziereinrichtung oder Ultrafiltrationseinrichtung verfügen, die der besseren Übersichtlichkeit halber jedoch nicht dargestellt sind.

Die erfindungsgemäße Vorrichtung zum Betreiben der Blutpumpe 6 wird bei dem vorliegenden Ausführungsbeispiel als Bestandteil der extrakorporalen Blutbehandlungsvorrichtung beschrieben, wobei die Überwachung des ordnungsgemäßen Betriebs der Blutpumpe möglich ist. Grundsätzlich kann aber auch der ordnungsgemäße Betrieb anderer beteiligter Pumpen, beispielsweise der Substituatpumpe, überwacht werden.

Auch wenn die Vorrichtung zum Betreiben einer elektrischen peristaltischen Schlauchpumpe im vorliegenden Ausführungsbeispiel in Verbindung mit einer extrakorporalen Blutbehandlungsvorrichtung beschrieben wird, kann die Vorrichtung zum Betreiben der Schlauchpumpe aber auch eine selbständige Baugruppe bilden, die bei sämtlichen medizin-technischen Einrichtungen zur Überwachung des ordnungsgemäßen Betriebs von Schlauchpumpen eingesetzt werden kann.

Nachfolgend wird die Funktionsweise der Vorrichtung zum Betreiben der Blutpumpe 6 im Einzelnen erläutert.

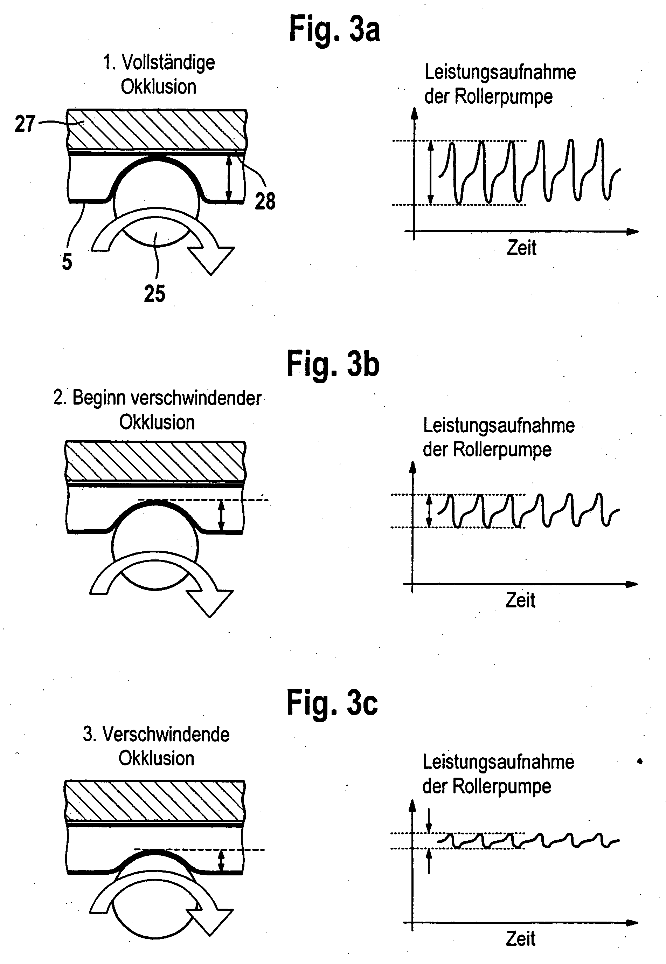

Bei der Blutpumpe 6 handelt es sich um eine Rollenpumpe. Da Rollenpumpen allgemein bekannt sind, erübrigt sich eine detaillierte Beschreibung. Die Figuren 3a bis 3c zeigen eine Prinzipdarstellung der Funktionsweise der Rollenpumpe, wobei nur eine der Rollen 25 dargestellt ist. Die Rollen 25 sind an einem nicht dargestellten Rotor drehbar gelagert. Die Schlauchleitung, im vorliegendem Ausführungsbeispiel die arterielle Blutleitung 5, befindet sich zwischen den Rollen 25 und einem Stator 27, der eine Rollenbahn 28 als Widerlager bildet.

Die Figuren 3a bis 3c zeigen nur eine Prinzipdarstellung. Bei den Rollenpumpen erstreckt sich die Rollenbahn bogenförmig um den mit Rollen besetzten Rotor. Die Rollen 25 sind an dem Rotor gegen die Rollenbahn 28 federnd vorgespannt, so dass die Rollen von der Schlauchleitung abheben können.

Figur 3a zeigt den Fall, dass die Rollen 25 die Schlauchleitung vollständig okkludieren, Figur 3b den Fall, dass die Rollen von der Schlauchleitung abzuheben beginnen und Figur 3c den Fall, dass die Schlauchleitung von den Rollen nicht mehr vollständig okkludiert wird, so dass die Blutpumpe nicht mehr ordnungsgemäß betrieben wird.

Die Überwachung des Betriebs der Blutpumpe beruht auf der Auswertung der von der Pumpe aufgenommenen Leistung. Die Leistungsaufnahme kann aus dem Produkt der an dem Gleichstrom-Elektromotor der Blutpumpe 6 anliegenden Spannung und des in den Motor fließenden Stroms berechnet werden. Es genügt aber auch eine mit der Leistung korrelierende Größe zu bestimmen. Da die Spannung als konstant angenommen werden kann, genügt es, nur den Motorstrom zu messen. Es sei bemerkt, dass die Blutpumpe grundsätzlich auch mit einem Wechselstrommotor betrieben werden kann.

Während einer extrakorporalen Blutbehandlung kann der Strömungswiderstand im Dialysator ansteigen (Klotting). Dies führt zu einem Anstieg des Drucks in der arteriellen Blutleitung 5 stromauf der Blutpumpe 6 und stromab des Dialysators 1, welcher aber mit dem venösen Drucksensor 24 nicht gemessen werden kann. Mit steigendem arteriellen Druck nimmt die Leistungsaufnahme der Blutpumpe 6 zu.

Eine Analyse der Leistungsaufnahme zeigt, dass die von der Pumpe aufgenommene Leistung oder eine mit der Leistung korrelierende physikalische Größe sowohl einen Gleichanteil, der sich nicht periodisch ändert, als auch einen sich periodisch ändernden Wechselanteil aufweist.

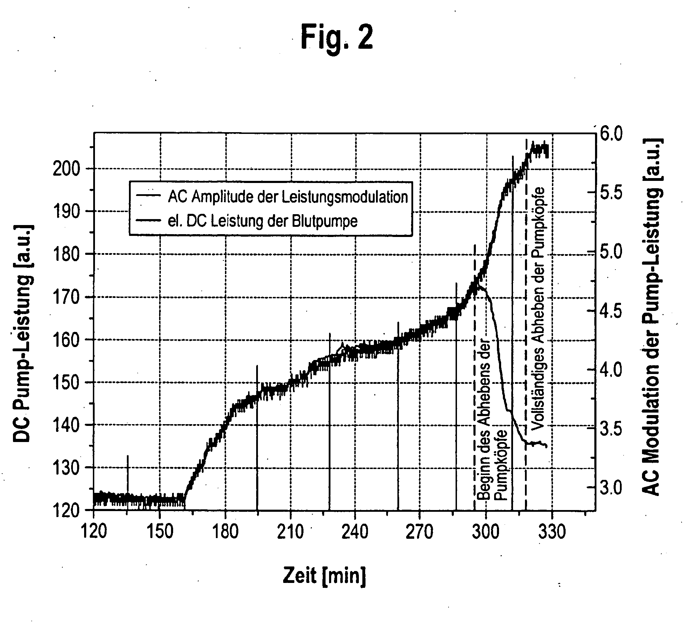

Figur 2 zeigt die Leistungsaufnahme der Blutpumpe als Funktion der Zeit in Bezug auf den Gleichanteil (DC) sowie den Wechselanteil (AC).

Die Größe der AC-Leistungsaumahme verläuft proportional mit der Größe der DC-Leistungsaufnahme, sofern die Blutpumpe vollständig okkludiert. Durch entsprechende Skalierung können die Kurven für diesen Fall zur Deckung gebracht werden. Heben die Rollen der Blutpumpe aber von dem Blutschlauchsegment ab, so dass die Förderrate der Pumpe nachlässt, verschwindet die Deckung. Dies ist in Figur 2 graphisch während einer in-vitro Dialysebehandlung mit Blutersatzstoff graphisch gezeigt. Während dieser Behandlung wurde durch exzessive Ultrafiltration der Blutersatzstoff bis zum Klotting des Dialysators eingedickt, so dass der Strömungswiderstand im Dialysator kontinuierlich stieg, bis die Widerstandskraft aufgrund des erhöhten Staudruckes die Rückstellkräfte der Rollen der Pumpe überschritten haben und die Okklusion zu verschwinden begann. Durch die Verringerung des Hubes der Pumpenrollen nimmt die Modulationsbreite der aufgenommenen Leistung des Motors ab, obwohl die mittlere Leistungsaufnahme aufgrund des hohen Strömungswiderstandes im Schlauchsegment zunimmt.

Die Figuren 3a bis 3c zeigen, dass die Amplitude der AC-Leistungsaufnahme mit dem Verschwinden der Okklusion abnimmt, während die DC-Leistungsaufnahme mit dem Verschwinden der Okklusion zunimmt.

Die erfindungsgemäße Blutbehandlungsvorrichtung arbeitet wie folgt:

Zur Vorbereitung der Dialysebehandlung stellt die Steuereinheit 16 einen Wert für die Blutflussrate $Q_b$ ein, der so klein ist, dass der Strömungswiderstand durch den Dialysator zu vernachlässigen ist. Der Druck in der arteriellen Blutleitung 5 stromauf des Dialysators 1 entspricht dann dem Druck, den der venöse Drucksensor 24 misst. Der Strom der Pumpe 6 wird nunmehr gemessen. Der gemessene Pumpenstrom $I_{p1}$ entspricht dem Druck $P_{ven}$.

$$\text{(Gleichung 1)} \qquad I(p=P_{ven}\ (Q_b \approx 0))=I_{p1}$$

Daraufhin schließt die Steuereinheit 16 das venöse Absperr-Ventil 12 sowie weitere der besseren Übersichtlichkeit halber nicht gezeigte Ventile in der Dialysierflüssigkeitszuführ- und -abführleitung 13, 14, so dass die Blutpumpe 6 jetzt gegen den Druck arbeitet, den die Rückstellkräfte der Rollen zu erzeugen in der Lage sind. Dieser Druck liegt technisch und normbedingt bei den in der Praxis eingesetzten Rollenpumpen bei etwa 1,6 bis 1,8 bar. Damit entspricht die Stromaufnahme der Blutpumpe einem Druck von 1,6 bis 1,8 bar.

$$\text{(Gleichung 2)} \qquad I(p=P_{Okk})=I_{p2}$$

Entsprechend dem linearen Zusammenhang zwischen Leistungsaufnahme und dem Druck p(I) stromauf des Dialysators kann der Druck aus der Stromaufnahme I bestimmt werden:

(Gleichung 3)

$$I_p(p) = \frac{I_{p2} - I_{p1}}{P_{Okk} - P_{ven}(Q_b = 0)} \left( p - P_{ven}(Q_b = 0) \right) + I_{p1}$$

$$\Rightarrow p(I) = \frac{P_{Okk} - P_{ven}(Q_b = 0)}{I_{p2} - I_{p1}} \left( I - I_{p1} \right) + P_{ven}(Q_b = 0)$$

Der Rechen- und Auswerteinheit 20 stehen die in Gleichung 3 angegebenen Größen zur Verfügung. Der venöse

Druck $P_{ven}$ wird mit dem venösen Druckmesser 24 gemessen, während die Blutpumpe 6 mit minimaler Förderrate betrieben wird, wobei der Strom $I_{p1}$ von der Steuereinheit 16 gemessen bzw. vorgegeben wird. Der Druck in der arteriellen Blutleitung 6 $P_{Okk}$, der abhängig vom Pumpentyp ist, wird mit einem Wert angenommen, der zwischen 1,6 und 1,8 bar liegt.

Während der Blutbehandlung wird der arterielle Druck in der arteriellen Blutleitung 5 fortlaufend von der Rechen- und Auswerteinheit 20 nach Gleichung 3 berechnet.

Darüber hinaus bestimmt die Rechen- und Auswerteinheit 20 während der Blutbehandlung aus der Leistungsaufnahme oder einer mit der Leistungsaufnahme korrelierenden physikalischen Größe, insbesondere aus dem Pumpenstrom, fortlaufend den Gleichanteil (DC-Leistungsaufnahme) und Wechselanteil (AC-Leistungsaufnahme). Für den Wechselanteil wird die sich aus dem Produkt von Rotorfrequenz und Rollenzahl sich ergebende Frequenz zu Grunde gelegt. Solange die Blutpumpe die Schlauchleitung vollständig okkludiert, ist der Quotient der zeitlichen Änderung der Amplitude der AC-Leistungsaufnahme $A_{AC}$ und der zeitlichen Änderung der DC-Leistungsaufnahme $I_{DC}$ konstant, d.h. die Steigungen der Amplitudenmodulation $A_{AC}$ und des Wertes der mittleren Gleichstromaufnahme $I_{DC}$ verlaufen in einem linearen Verhältnis zueinander.

$$(\text{Gleichung 4}) \qquad \frac{\frac{d}{dt}A_{AC}}{\frac{d}{dt}I_{DC}} = \frac{dA_{AC}}{dI_{DC}} = const,$$

wobei mit T als Periodizität der Blutpumpe normiert auf die Anzahl der Rollen und $\varphi$ als Normierung der Phasenlage, d.h. die Phasenschiebung zwischen der Modulation $A_{AC}$ und der im Integrationsintervall verwendeten trigonometrischen Funktion sin(x), gilt:

$$(\text{Geichung 5}) \qquad I_{DC} = \frac{1}{T}\int_{T} I(t)\,dt$$

$$(\text{Gleichung 6}) \qquad A_{AC} = \frac{1}{T}\int_{T} I(t)\sin(2\pi \tfrac{t}{T} + \varphi)\,dt$$

Wenn das in Gleichung 4 angegebene Verhältnis nicht mehr konstant ist, sondern gegen Null zu tendieren beginnt, nimmt die Okklusion der Blutpumpe ab, d.h. die Rollen der Blutpumpe heben von dem Schlauchsegment ab. Dies ist insbesondere dann der Fall, wenn das Verhältnis ein negatives Vorzeichen bekommt.

$$(\text{Gleichung 7}) \qquad \frac{\frac{d}{dt}A_{AC}}{\frac{d}{dt}I_{DC}} = \frac{dA_{AC}}{dI_{DC}} \leq 0$$

Bei vollständiger Aufhebung der Okklusion gilt dann:

$$(\text{Gleichung 8}) \qquad \frac{d}{dt} I_{DC} = 0,$$

$$(\text{Gleichung 9}) \qquad \frac{d}{dt} A_{AC} = 0$$

Die Rechen- und Auswerteinheit 20 berechnet fortlaufend während der Behandlung den Quotienten der zeitlichen Änderung der AC-Leistungsaufnahme $A_{AC}$ und der DC-Leistungsaufnahme $I_{DC}$ nach Gleichung 4. Zur Durchführung der erforderlichen Rechenoperationen, insbesondere zur Bildung der Differentiale verfügt die Rechen- und Auswerteinheit 20 über einen Mikroprozessor.

Den ermittelten Quotienten vergleicht die Rechen- und Auswerteinheit 20 mit vorgegebenen Grenzwerten. Für den Fall, dass der ermittelte Quotient größer als ein erster vorgegebener Grenzwert ist, d.h. konstant ist (Gleichung 4), wird angenommen, dass die Blutpumpe 6 die arterielle Blutleitung 5 vollständig okkludiert (Fig. 3a). Wenn aber der erste Grenzwert unterschritten wird, erzeugt die Rechen- und Auswerteinheit 20 ein erstes Alarmsignal, so dass die Alarmeinheit 23 einen ersten optischen und/oder akustischen Alarm gibt, der signalisiert, dass die Rollen der Blutpumpe von der Schlauchleitung abzuheben beginnen (Fig. 3b). Wird ein weiterer zweiter Grenzwert, der kleiner als der erste Grenzwert ist, unterschritten, erzeugt die Rechen- und Auswerteinheit ein zweites Alarmsignal, so dass die Alarmeinrichtung einen zweiten Alarm gibt, der signalisiert, dass die Blutpumpe die Schlauchleitung nicht mehr vollständig okkludiert (Fig. 3c). Dann ist ein ordnungsgemäßer Betrieb der Schlauchpumpe nicht mehr gegeben, da der Druck in der arteriellen Blutleitung 5 aufgrund einer Erhöhung des Strömungswiderstandes im Dialysator 1 einen vorgegebenen Grenzwert überschritten hat.

Eine alternative Ausführungsform sieht vor, den Quotienten nicht mit einem Grenzwert zu vergleichen, sondern die zeitliche Änderung des Gleich- und Wechselanteils der Leistungsaufnahme in vorgegebenen Zeitintervallen, d.h. das Maß des Ansteigens (Steigung) der Größen logisch miteinander wie folgt zu verknüpfen.

Wenn sowohl der Gleichanteil als auch der Wechselanteil der Leistungsaufnahme ansteigt, wird darauf geschlossen, dass der arterielle Druck ansteigt. Wenn nur der Gleichanteil der Leistungsaufnahme ansteigt, der Wechselanteil der Leistungsaufnahme jedoch stagniert, wird darauf geschlossen, dass die Okklusion der Blutpumpe abzunehmen beginnt. Wenn der Wechselanteil der Leistungsaufnahme bei ansteigendem Gleichanteil der Leistungsaufnahme zu sinken beginnt, wird darauf geschlossen, dass die Rollen der Blutpumpe von der Schlauchleitung abgehoben haben. Für den Fall, dass nach dem Abheben der Pumpenrollen sowohl Gleich- als auch Wechselanteil der Leistungsaufnahme stagnieren, stellt die Rechen- und Auswerteinheit 20 fest, dass die Blutpumpe 6 nicht mehr fördert.

Grundsätzlich ist es auch möglich, beide Auswertverfahren miteinander zu verknüpfen, wobei sowohl das Über- bzw. Unterschreiten vorgegebener Grenzwerte als auch eine logische UND-Verknüpfung des Gleich- und Wechselanteils der Leistungsaufnahme Berücksichtigung finden.

Es ist auch möglich, nur den Quotienten von Gleichanteil und Wechselanteil zu überwachen. Wie aus Fig. 2 ersichtlich ist, bleibt dieser Quotient in einem gewissen Bereich konstant, solange die Pumpe nicht okkludierend arbeitet. Die Abweichung der Kurvenform zu einem späteren Zeitpunkt führt jedoch zu einer Veränderung (Verkleinerung) des Quotienten, die als Störfall detektiert wird.

**Patentansprüche**

1. Verfahren zum Betreiben einer elektrischen peristaltischen Schlauchpumpe (6) zur Förderung einer Flüssigkeit in einer Schlauchleitung (5, 7), bei dem während des Betriebs der Pumpe (6) die von der Pumpe aufgenommene Leistung oder eine mit der Leistung korrelierende physikalische Größe bestimmt wird, **dadurch gekennzeichnet, dass** aus der gemessenen physikalischen Größe ein Gleichanteil (DC), der sich nicht periodisch ändert, und ein dem Gleichanteil überlagerter Wechselanteil (AC) bestimmt werden, der sich periodisch ändert, wobei auf der Grundlage des Gleichanteils und des Wechselanteils während des Betriebs der Schlauchpumpe auf einen nicht ordnungsgemäßen Betrieb der Schlauchpumpe geschlossen wird.

2. Verfahren nach Anspruch 1, **dadurch gekennzeichnet, dass** während des Betriebs der Schlauchpumpe die zeitliche Änderung des Gleichanteils (d/dt $I_{DC}$) und die zeitliche Änderung des Wechselanteils (d/dt $A_{AC}$) in vorgege-

EP 1 994 284 B1

benen Zeitintervallen zueinander in ein Verhältnis gesetzt werden, wobei auf der Grundlage der zeitlichen Änderung des ermittelten Verhältnisses zwischen der zeitlichen Änderung des Gleichanteils und Wechselanteils in vorgegebenen Zeitintervallen während des Betriebs der Schlauchpumpe auf einen nicht ordnungsgemäßen Betrieb der Schlauchpumpe geschlossen wird.

3. Verfahren nach Anspruch 2, **dadurch gekennzeichnet, dass** der Quotient ($dA_{AC}/d\,I_{DC}$) aus der zeitlichen Änderung des Wechselanteils (d/dt $A_{AC}$) und des Gleichanteils (d/dt $I_{DC}$) in vorgegebenen Zeitintervallen berechnet und mit einem vorgegebenen Grenzwert verglichen wird, wobei auf eine nicht ausreichende Okklusion der Schlauchpumpe geschlossen wird, wenn der Quotient den vorgegebenen Grenzwert unterschreitet.

4. Verfahren nach Anspruch 1, **dadurch gekennzeichnet, dass** der Quotient ($A_{AC}/\,I_{DC}$) des Wechselanteils ($A_{AC}$) und des Gleichanteils ($I_{DC}$) in vorgegebenen Zeitintervallen berechnet und mit einem vorgegebenen Grenzwert verglichen wird, wobei auf eine nicht ausreichende Okklusion der Schlauchpumpe geschlossen wird, wenn der Quotient den vorgegebenen Grenzwert unterschreitet.

5. Verfahren nach einem der Ansprüche 1 bis 4, **dadurch gekennzeichnet, dass** die Schlauchpumpe eine okklusive Rollenpumpe ist, wobei die Schlauchleitung zwischen einem Stator, der eine Rollenbahn als Widerlager bildet, und einem mit drehbar gelagerten Rollen besetzten Rotor angeordnet ist.

6. Verfahren nach einem der Ansprüche 1 bis 5, **dadurch gekennzeichnet, dass** aus der mit der Leistung korrelierenden physikalischen Größe der Druck stromab der Schlauchpumpe bestimmt wird.

7. Verfahren nach einem der Ansprüche 1 bis 6, **dadurch gekennzeichnet, dass** eine elektrische peristaltische Schlauchpumpe einer extrakorporalen Blutbehandlungsvorrichtung betrieben wird.

8. Verfahren nach einem der Ansprüche 1 bis 7, **dadurch gekennzeichnet, dass** die peristaltische Schlauchpumpe in einer arteriellen Schlauchleitung angeordnet ist, die zu einer Blutbehandlungseinheit führt, von der eine venöse Schlauchleitung abgeht, wobei die arterielle und venöse Schlauchleitung zusammen mit der Blutbehandlungseinheit einen extrakorporalen Blutkreislauf bilden.

9. Vorrichtung zum Betreiben einer elektrischen peristaltischen Schlauchpumpe (6) zur Förderung einer Flüssigkeit in einer Schlauchleitung (5, 7), mit
Mitteln (16,20) zum Bestimmen der während des Betriebs der Pumpe von der Pumpe aufgenommenen Leistung oder einer mit der Leistung korrelierenden physikalischen Größe,
**dadurch gekennzeichnet, dass** die Vorrichtung aufweist:

Mittel (16, 20) zum Bestimmen eines Gleichanteils (DC), der sich nicht periodisch ändert, und eines Wechselanteils (AC), der sich periodisch ändert, aus der gemessenen physikalischen Größe, und
Mittel (16, 20) zur Feststellung eines nicht ordnungsgemäßen Betriebs der Schlauchpumpe, die derart ausgebildet sind, dass auf der Grundlage des Gleichanteils (DC) und des Wechselanteils (AC) während des Betriebs der Schlauchpumpe auf einen nicht ordnungsgemäßen Betrieb der Schlauchpumpe geschlossen wird.

10. Vorrichtung nach Anspruch 9, **dadurch gekennzeichnet, dass** die Mittel (16, 20) zur Feststellung eines nicht ordnungsgemäßen Betriebs der Schlauchpumpe derart ausgebildet sind, dass die zeitliche Änderung des Gleichanteils (d/dt $I_{DC}$) und die zeitliche Änderung des Wechselanteil (d/dt $A_{AC}$) in vorgegebenen Zeitintervallen während des Betriebs der Schlauchpumpe zueinander in ein Verhältnis gesetzt werden, wobei auf der Grundlage der zeitlichen Änderung des ermittelten Verhältnisses zwischen der zeitlichen Änderung des Gleichanteils und Wechselanteils in vorgegebenen Zeitintervallen während des Betriebs der Schlauchpumpe auf einen nicht ordnungsgemäßen Betrieb der Schlauchpumpe geschlossen wird.

11. Vorrichtung nach Anspruch 10, **dadurch gekennzeichnet, dass** die Mittel (16,20) zu Feststellung eines nicht ordnungsgemäßen Betriebs der Schlauchpumpe derart ausgebildet sind, dass der Quotient ($dA_{AC}/d\,I_{DC}$) aus der zeitlichen Änderung des Wechselanteils (d/dt $A_{AC}$) und des Gleichanteils (d/dt $I_{DC}$) in vorgegebenen Zeitintervallen während des Betriebs der Schlauchpumpe berechnet und mit einem vorgegebenen Grenzwert verglichen wird, wobei auf eine nicht ausreichende Okklusion der Schlauchpumpe geschlossen wird, wenn der Quotient den vorgegebenen Grenzwert unterschreitet.

12. Vorrichtung nach Anspruch 9, **dadurch gekennzeichnet, dass** die Mittel (16, 20) zur Feststellung eines nicht ordnungsgemäßen Betriebs der Schlauchpumpe derart ausgebildet sind, dass der Quotient ($A_{AC}$/ $I_{DC}$) des Wechselanteils ($A_{AC}$) und des Gleichanteils ($I_{DC}$) in vorgegebenen Zeitintervallen während des Betriebs der Schlauchpumpe berechnet und mit einem vorgegebenen Grenzwert verglichen wird, wobei auf eine nicht ausreichende Okklusion der Schlauchpumpe geschlossen wird, wenn der Quotient den vorgegebenen Grenzwert unterschreitet.

**13.** Vorrichtung nach einem der Ansprüche 9 bis 12, **dadurch gekennzeichnet, dass** die Schlauchpumpe eine okklusive Rollenpumpe (6) ist, wobei die Schlauchleitung zwischen einem Stator, der eine Rollenbahn als Widerlager bildet, und einem mit drehbar gelagerten Rollen besetzten Rotor angeordnet ist.

**14.** Vorrichtung nach einem der Ansprüche 9 bis 13, **dadurch gekennzeichnet, dass** die Vorrichtung Mittel (13) zum Bestimmen des Drucks stromab der Schlauchpuinpe aufweist, die derart ausgebildet sind, dass aus der mit der Leistung korrelierenden physikalischen Größe der Druck stromab der Schlauchpumpe bestimmt wird.

**15.** Blutbehandlungsvorrichtung mit einer elektrischen peristaltischen Schlauchpumpe (6), **dadurch gekennzeichnet, dass** die Blutbehandlungsvorrichtung eine Vorrichtung nach einem der Ansprüche 9 bis 14 zum Betreiben der peristaltischen Schlauchpumpe (6) aufweist.

**16.** Blutbehandlungsvorrichtung nach Anspruch 15, **dadurch gekennzeichnet, dass** die Blutbehandlungsvorrichtung eine Blutbehandlungseinheit (1) aufweist, zu der eine arterielle Schlauchleitung (5) führt und von der eine venöse Schlauchleitung (7) abgeht, wobei die elektrische peristaltische Schlauchpumpe (6) in der arteriellen Schlauchleitung (5) angeordnet ist.

**Claims**

**1.** A method for the operation of an electric peristaltic hose pump (6) for conveying a fluid in a hose line (5, 7), wherein the power consumed by the pump or a physical magnitude correlating with the power is determined during the operation of the pump (6), **characterised in that** a direct component (DC) that does not change periodically and an alternating component (AC) that changes periodically and is superimposed on the direct component are determined from the measured physical magnitude, whereby it is concluded that there is an incorrect operation of the hose pump on the basis of the direct component and the alternating component during the operation of the hose pump.

**2.** The method according to claim 1, **characterised in that**, during the operation of the hose pump, the time-related change in the direct component (d/dt $I_{DC}$) and the time-related change in the alternating component (d/dt $A_{AC}$) in preset time intervals are put into a relationship with one another, whereby it is concluded that there is an incorrect operation of the hose pump on the basis of the time-related change in the ascertained relationship between the time-related change in the direct component and alternating component in preset time intervals during the operation of the hose pump.

**3.** The method according to claim 2, **characterised in that** the quotient ($dA_{AC}$/d$I_{DC}$) of the time-related change in the alternating component (d/dt $A_{AC}$) and the direct component (d/dt$I_{DC}$) is calculated in preset time intervals and compared with a preset limiting value, whereby it is concluded that there is an inadequate occlusion of the hose pump if the quotient falls below the preset limiting value.

**4.** The method according to claim 1, u̲ the quotient ($A_{AC}$/$I_{DC}$) of the alternating component ($A_{AC}$) and the direct component ($I_{DC}$) is calculated in preset time intervals and compared with a preset limiting value, whereby it is concluded that there is an inadequate occlusion of the hose pump if the quotient falls below the preset limiting value.

**5.** The method according to any one of claims 1 to 4, **characterised in that** the hose pump is an occlusive roller pump, whereby the hose line is inserted between a stator, which forms a roller path as a counter-bearing, and a rotor which is fitted with rollers mounted rotatably.

**6.** The method according to any one of claims 1 to 5, **characterised in that** the pressure downstream of the hose pump is determined from the physical magnitude correlating with the power.

**7.** The method according to any one of claims 1 to 6, **characterised in that** an electric peristaltic hose pump of an extracorporeal blood treatment apparatus is operated.

8. The method according to any one of claims 1 to 7, **<u>characterised in that</u>** the peristaltic hose pump is arranged in an arterial hose line, which leads to a blood treatment unit, from which a venous hose line departs, whereby the arterial and venous hose line together with the blood treatment unit form an extracorporeal blood circuit.

9. A device for the operation of an electric peristaltic hose pump (6) for conveying a fluid in a hose line, (5, 7) with
means (16, 20) for determining the power consumed by the pump during the operation of the pump or a physical magnitude correlating with the power,
**characterised in that** the device has:

means (16, 20) for determining from the measured physical magnitude a direct component (DC) which does not change periodically and an alternating component (AC) which does change periodically, and
means (16, 20) for determining an incorrect operation of the hose pump, which are designed in such a way that it is concluded that there is an incorrect operation of the hose pump on the basis of the direct component (DC) and the alternating component (AC) during the operation of the hose pump.

10. The device according to claim 9, **characterised in that** the means (16,20) for ascertaining an incorrect operation of the hose pump are designed in such a way that the time-related change in the direct component (d/dt $I_{DC}$) and the time-related change in the alternating component (d/dt $A_{AC}$) in preset time intervals during the operation of the hose pump are put into a relationship with one another, whereby it is concluded that there is an incorrect operation of the hose pump on the basis of the time-related change in the ascertained relationship between the time-related change in the direct component and alternating component in preset time intervals during the operation of the hose pump.

11. The device according to claim 10, **<u>characterised in that</u>** the means (16, 20) for ascertaining an incorrect operation of the hose pump are designed in such a way that the quotient ($dA_{AC}/dI_{DC}$) of the time-related change in the alternating component (d/dt $A_{AC}$) and the direct component (d/dt $I_{DC}$) is calculated in preset time intervals during the operation of the hose pump and compared with a preset limiting value, whereby it is concluded that there is an inadequate occlusion of the hose pump if the quotient falls below the preset limiting value.

12. The device according to claim 9, **<u>characterised in that</u>** the means (16, 20) for ascertaining an incorrect operation of the hose pump are designed in such a way that the quotient ($A_{AC}/I_{DC}$) of the alternating component ($A_{AC}$) and the direct component ($I_{DC}$) is calculated in preset time intervals during the operation of the hose pump and compared with a preset limiting value, whereby it is concluded that there is an inadequate occlusion of the hose pump if the quotient falls below the preset limiting value.

13. The device according to any one of claims 9 to 12, **<u>characterised in that</u>** the hose pump is an occlusive roller pump (6), whereby the hose line is arranged between a stator, which forms a roller path as a counter-bearing, and a rotor which is fitted with rollers mounted in a rotatably.

14. The device according to any one of claims 9 to 13, **<u>characterised in that</u>** the device has means (13) for determining the pressure downstream of the hose pump, which are designed in such a way that the pressure downstream of the hose pump is determined from the physical magnitude correlating with the power.

15. A blood treatment apparatus with an electric peristaltic hose pump (6), **<u>characterised in that</u>** the blood treatment apparatus has a device according to any one of claims 9 to 14 for the operation of the peristaltic hose pump (6).

16. The blood treatment apparatus according to claim 15, **<u>characterised in that</u>** the blood treatment apparatus has a blood treatment unit (1), to which an arterial hose line (5) leads and from which a venous hose line (7) departs, whereby the electric peristaltic hose pump (6) is arranged in the arterial hose line (5).

**Revendications**

1. Procédé pour actionner une pompe péristaltique électrique (6) pour acheminer un fluide dans une conduite tubulaire (5, 7) dans lequel, pendant le fonctionnement de la pompe (6), le débit absorbé par la pompe ou une grandeur physique corrélée au débit est déterminé(e), **caractérisé en ce que**, à partir de la grandeur physique mesurée, une composante continue (DC) qui ne se modifie pas périodiquement, et une composante alternative (AC) superposée à la composante continue, qui se modifie périodiquement, sont déterminées, dans lequel on conclut sur la base de la composante continue et de la composante alternative pendant le fonctionnement de la pompe péristaltique, à un fonctionnement anormal de la pompe péristaltique.

2. Procédé selon la revendication 1, **caractérisé en ce que**, pendant le fonctionnement de la pompe péristaltique, la modification temporelle de la composante continue (d/dt $I_{DC}$) et la modification temporelle de la composante alternative (d/dt $A_{AC}$) sont en rapport à intervalles temporels prédéter-minés, dans lequel on conclut sur la base de la modification temporelle du rapport déterminé entre la modification temporelle de la composante conti-nue et de la composante alternative, à intervalles temporels prédéterminés pendant le fonctionnement de la pompe péristaltique, à un fonctionnement anormal de la pompe péristaltique.

3. Procédé selon la revendication 2, **caractérisé en ce que** le quotient (d$A_{AC}$/d $I_{DC}$) de la modifi-cation temporelle de la composante alternative (d/dt $A_{AC}$) et de la composante continue (d/dt $I_{DC}$) est calculé à intervalles temporels prédéterminés et est comparé à une valeur seuil prédéterminée, dans lequel on conclut à une occlusion insuffi-sante de la pompe péristaltique lorsque le quotient dépasse la valeur seuil prédéterminée.

4. Procédé selon la revendication 1, **caractérisé en ce que** le quotient ($A_{AC}$/$I_{DC}$) de la composante alternative ($A_{AC}$) et de la composante continue ($I_{DC}$) est calculé à intervalles temporels prédéter-minés et est comparé à une valeur seuil prédéter-minée, dans lequel on conclut à une occlusion insuffisante de la pompe péristaltique lorsque le quotient est inférieur à la valeur seuil prédé-terminée.

5. Procédé selon l'une des revendications 1 à 4, **caractérisé en ce que** la pompe péristaltique est une pompe à rouleaux occlusive, la conduite tubu-laire étant disposée entre un stator qui forme un transporteur à rouleaux comme contre-palier et un rotor équipé de rouleaux montés de façon à pou-voir tourner.

6. Procédé selon l'une des revendications 1 à 5, **caractérisé en ce que**, à partir de la grandeur physique corrélée au débit, la pression est déterminée en aval de la pompe péristaltique.

7. Procédé selon l'une des revendications 1 à 6, **caractérisé en ce qu'**une pompe péristaltique électrique d'un dispositif de traitement sanguin extracorporel est actionnée.

8. Procédé selon l'une des revendications 1 à 7, **caractérisé en ce que** la pompe péristaltique est disposée dans une conduite tubulaire artérielle qui conduit à une unité de traitement du sang de laquelle part une conduite tubulaire veineuse, les conduites tubulaires artérielle et veineuse formant ensemble avec l'unité de traitement du sang, une circulation sanguine extracorporelle.

9. Dispositif d'actionnement d'une pompe péris-taltique électrique (6) pour acheminer un fluide dans une conduite tubulaire (5, 7) comportant
des moyens (16, 20) de détermination du débit absorbé par la pompe pendant le fonctionnement de la pompe ou une grandeur physique corrélée au débit,
**caractérisé en ce que** le dispositif présente:

- des moyens (16, 20) de détermination d'une com-posante continue (DC) qui ne se modifie pas périodiquement, et d'une composante alternative (AC) qui se modifie périodiquement, à partir de la grandeur physique mesurée, et
- des moyens (16, 20) de constatation d'une fonc-tionnement anormal de la pompe péristaltique qui sont conçus de telle sorte que, sur la base de la composante continue (DC) et de la composante alternative (AC) pendant le fonctionnement de la pompe péristaltique, on conclut à un fonction-nement anormal de la pompe péristaltique.

10. Dispositif selon la revendication 9, caracté-risé en ce que les moyens (16, 20) de constata-tion d'un fonctionnement anormal de la pompe sont conçus de telle sorte que la modification temporelle de la composante continue (d/dt $I_{DC}$) et la modification temporelle de la composante alterna-tive (d/dt $A_{AC}$) à intervalles temporels prédéter-minés pendant le fonctionnement de la pompe péristaltique sont en rapport l'une avec l'autre, dans lequel on conclut sur la base de la modifi-cation temporelle du rapport déterminé entre la modification temporelle de la composante continue et de la composante alternative, à intervalles prédéterminés pendant le fonctionnement de la pompe péristaltique, à un fonctionnement anormal de la pompe péristaltique.

11. Dispositif selon la revendication 10, carac-térisé en ce que les moyens (16, 20) de constata-tion d'un fonctionnement anormal de la pompe péristaltique sont conçus de telle sorte que le quotient (d$A_{AC}$/d $I_{DC}$) de la modification tempo-relle de la composante alternative (d/dt $A_{AC}$) et de la composante continue (d/dt $I_{DC}$) est calculé à intervalles temporels prédéterminés pendant le fonctionnement de la pompe péristaltique et est comparé à une valeur seuil prédéterminée, dans lequel on conclut à une occlusion insuffisante de la pompe péristaltique lorsque le quotient dépasse la valeur seuil prédéterminée.

12. Dispositif selon la revendication 9, carac-térisé en ce que les moyens (16, 20) de constata-tion d'un fonctionnement anormal de la pompe péristaltique sont conçus de telle sorte que le quotient ($A_{AC}/I_{DC}$) de la composante alternative ($A_{AC}$) et de la composante continue ($I_{DC}$) est cal-culé à intervalles temporels prédéterminés pendant le fonction-nement de la pompe péristaltique et est comparé à une valeur seuil prédéterminée, dans lequel on conclut à une occlusion insuf-fisante de la pompe péristaltique lorsque le quotient est inférieur à la valeur seuil prédé-terminée.

13. Dispositif selon l'une des revendications 9 à 12, **caractérisé en ce que** la pompe péristaltique est une pompe à rouleaux occlusive (6), la con-duite tubulaire étant disposée entre un stator qui forme un transporteur à rouleaux comme contre-palier et un rotor équipé de rouleaux montés de façon à pouvoir tourner.

14. Dispositif selon l'une des revendications 9 à 13, **caractérisé en ce que** le dispositif présente des moyens (13) pour déterminer la pression en aval de la pompe péristaltique qui est conçue de telle sorte qu'à partir de la grandeur physique corrélée au débit, la pression est déterminée en aval de la pompe péristaltique.

15. Dispositif de traitement du sang avec une pompe péristaltique. électrique (6), **caractérisé en ce que** le dispositif de traitement du sang présente un dispositif selon l'une des revendications 9 à 14 pour faire fonctionner la pompe péristaltique (6).

16. Dispositif de traitement du sang selon la revendication 15, **caractérisé en ce que** le dispositif de traitement du sang présente une unité de traitement du sang (1) à laquelle conduit une conduite tubulaire artérielle (5) et de laquelle part une conduite tubulaire veineuse (7), la pompe péristaltique électrique (6) étant disposée dans la conduite tubulaire artérielle (5).

# Fig. 1

# Fig. 2

# Fig. 3a

### 1. Vollständige
### Okklusion

27

28

5

25

Leistungsaufnahme
der Rollerpumpe

Zeit

# Fig. 3b

### 2. Beginn verschwindender
### Okklusion

Leistungsaufnahme
der Rollerpumpe

Zeit

# Fig. 3c

### 3. Verschwindende
### Okklusion

Leistungsaufnahme
der Rollerpumpe

Zeit

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 5629871 A
- US 4781525 A
- WO 9745150 A

**In der Beschreibung aufgeführte Nicht-Patentliteratur**

- Gesellschaft für angewandte Medizintechnik. Dialysetechnik. m.b.H. & Co, 1988